(19) Europäisches Patentamt
European Patent Office
Office européen des brevets

(11) Veröffentlichungsnummer : **0 045 853**
**B1**

(12) # EUROPÄISCHE PATENTSCHRIFT

(45) Veröffentlichungstag der Patentschrift : **02.05.84**

(51) Int. Cl.³ : **B 01 J 23/88**, B 01 J 27/18, C 07 C 51/25, C 07 C 57/04

(21) Anmeldenummer : **81105341.2**

(22) Anmeldetag : **09.07.81**

(54) **Oxidationskatalysator insbesondere für die Herstellung von Methacrylsäure durch Gasphasenoxidation von Methacrolein.**

(30) Priorität : **09.08.80 DE 3030243**

(43) Veröffentlichungstag der Anmeldung : **17.02.82 Patentblatt 82/07**

(45) Bekanntmachung des Hinweises auf die Patenterteilung : **02.05.84 Patentblatt 84/18**

(84) Benannte Vertragsstaaten :
**DE FR GB**

(56) Entgegenhaltungen :
**DE-A- 2 523 757**
**DE-A- 2 952 455**
**GB-A- 2 001 255**
**US-A- 4 049 574**
**US-A- 4 202 826**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Krabetz, Richard, Dr.**
**Unterer Waldweg 8**
**D-6719 Kirchheim (DE)**
Erfinder : **Merger, Franz, Dr.**
**Max-Slevogt-Strasse 25**
**D-6710 Frankenthal (DE)**
Erfinder : **Schwarzmann, Matthias, Dr.**
**Carl-Bosch-Strasse 54**
**D-6703 Limburgerhof (DE)**

**EP 0 045 853 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Oxidationskatalysator insbesondere für die Herstellung von Methacrylsäure durch Gasphasenoxidation von Methacrolein

Es ist bekannt, Methacrolein in der Gasphase mit sauerstoffenthaltenden Gasen zu Methacrylsäure zu oxidieren. Als Katalysatoren werden Gemische oder Verbindungen von Metalloxiden vorgeschlagen, die neben Molybdän, Phosphor und Alkalimetallen oder Thallium zusätzlich andere Metalle enthalten. Aus der jap. Patentveröffentlichung 68 122/1977 sind beispielsweise Katalysatoren bekannt, die zusätzlich Niob oder Vanadium und ein oder mehrere Elemente aus der Gruppe Mn, Sb, V, Fe, Cu, Ni, Si enthalten. Diese Katalysatoren benötigen jedoch Badtemperaturen über 330 °C und weisen daher eine kurze Lebensdauer auf.

In der jap. Patentveröffentlichung 122317/1977 werden Katalysatoren empfohlen, die als zusätzliche Elemente Rhodium, Metalle aus der Gruppe V, Cr, Fe und Metalle aus der Gruppe Se, Ti, Sn, W, Al, Th enthalten. Mit diesen Katalysatoren werden zwar anfänglich und kurzzeitig Selektivitäten von 87,7 Mol.% bei Badtemperaturen unter 300 °C erreicht. Es zeigte sich jedoch, daß bei der für den technischen Einsatz notwendigen Verformung der Katalysatormasse die Selektivität und Aktivität abnimmt und daß der Katalysator empfindlich ist gegen Schwankungen der Betriebsbedingungen, so daß die Lebensdauer insbesondere bei Umsätzen über 70 Mol.% zu wünschen übrig läßt.

Ein besonderer Nachteil der bisher bekannten Katalysatorsysteme besteht darin, daß ihre Leistung stark nachläßt, wenn technisches Methacrolein eingesetzt wird, das durch Kondensation von Propionaldehyd mit Formaldehyd oder Aminalen hergestellt ist und das in geringer Konzentration Verunreinigungen, wie organische Amine, Dimere des Methacroleins und Methylpentenal enthält.

Schließlich ist es aus der DE-A-2 523 757 bekannt, Acrylsäure oder Methacrylsäure durch katalytische Oxidation der entsprechenden ungesättigten Aldehyde mit Sauerstoff bei erhöhter Temperatur in der Gasphase herzustellen, wobei ein Katalysator der allgemeinen Formel $P_aMo_bCu_cAs_dX_eY_fO_g$ eingesetzt wird, bei dem a = 0,025 bis 1, b = 1, c = 0,002 5 bis 0,5, d = 0 bis 0,015, jedoch d $\neq$ 0,015, e = 0,002 5 bis 1, f = 0,003 bis 0,417 bedeuten, und g einen Wert hat, der vom Oxidationszustand des Katalysators abhängt. Als Bestandteil X kann der Katalysator unter anderem Vanadin, als Bestandteil Y unter anderem Cäsium enthalten. Die Aktivität und Selektivität derartiger Katalysatoren läßt jedoch noch zu wünschen übrig.

Es wurde nun gefunden, daß die Oxidation von Methacrolein zu Methacrylsäure in der Gasphase mit sauerstoffenthaltenden Gasen vorteilhaft durchgeführt werden kann, wenn Katalysatoren eingesetzt werden, deren Zusammensetzung durch die Formel

$$Mo_{12}P_aRh_bCu_cV_dCs_eX_fY_gZ_hO_x$$

wiedergegeben wird, wobei

X für Cr und/oder Fe
Y für Nb
Z für Na, Li, K und/oder Rb,
a für 0,1-4, vorzugsweise für 0,5-3,
b für 0,001-1, vorzugsweise für 0,02-0,5,
c für 0,05-2, vorzugsweise für 0,1-1,
d für 0,05-4, vorzugsweise für 0,1-3,
e für 0,1-5, vorzugsweise für 0,5-3,
f für 0-2, vorzugsweise für 0,01-2 wenn c 0,5,
g für 0-3, vorzugsweise für 0,1-2,
h für 0-2 und
x für die Anzahl der zur Absättigung der Valenzen der anderen Bestandteile erforderlichen Sauerstoffatome

stehen.

Die erfindungsgemäßen Katalysatoren sind besonders geeignet für die Umsetzung von Methacrolein, das durch Kondensation von Propionaldehyd und Formaldehyd oder Aminalen erhalten wird, da sie relativ unempfindlich gegen Verunreinigungen des Methacroleins wie Amine, Dimere des Methacroleins und Methylpentenal sind. Auch mit technischen Methacroleinqualitäten erreichen sie Selektivitäten zwischen 80 und 90 Mol.% bei Umsätzen über 60 Mol.% und Reaktionstemperaturen unter 330 °C, wobei auch nach Betriebszeiten von über 1 000 Stunden kein Nachlassen der Aktivität zu beobachten ist.

Ein weiterer Vorzug der erfindungsgemäßen Katalysatoren ist die gute Verformbarkeit der Katalysatormasse ohne nennenswerte Beeinträchtigung der katalytischen Eigenschaften. In manchen Fällen kann sogar eine Verbesserung der katalytischen Eigenschaften durch die Verformung der Katalysatormasse beobachtet werden. Die neuen Katalysatoren können in verschiedener Weise hergestellt, verformt und ggf. auf Träger aufgebracht werden. Ein einfaches Verfahren der Herstellung besteht darin, daß die Salze oder Oxide der Komponenten in wäßriger Lösung oder Suspension gemischt, anschließend bei Temperaturen zwischen 70 und 130 °C getrocknet und schließlich bei Temperaturen zwischen 180 und 600 °C calciniert werden. Es kann von Vorteil sein und wird daher vorgezogen, daß die Katalysatormasse ggf. nach einer verhältnismäßig niederen Temperatur z. B. bei 220 bis 340 °C durchgeführten Umwandlung der Salze in die Oxide, bei Temperaturen zwischen 350 und 600 °C, vorzugsweise bei 360 bis 500 °C, thermisch aktiviert werden. Vorzugsweise wird die Calcinierung in Abwesenheit einer Gasströmung

und/oder in Gegenwart von gasförmigem Ammoniak durchgeführt. Der Katalysator kann ohne oder mit üblichen Trägerstoffen, wie Kieselsäure, $Al_2O_3$, Metallsilikaten, $TiO_2$, $ZrO_2$ oder Bims vermischt eingesetzt werden.

Die Verformung kann vor oder nach der aktivierenden Calcinierung durchgeführt werden. Bei einer Tablettierung werden vorteilhaft Gleitmittel, z. B. Graphitpulver, in Mengen zwischen meist 1 und 5 Gew.% zugegeben, eine (eher schädliche) Befeuchtung des zu tablettierenden Pulvers, ist im allgemeinen nicht notwendig. Die Katalysatormasse kann auch zu Kugeln verformt werden, indem in bekannter Weise das Katalysatorpulver und ein flüssiges Bindemittel in einem bestimmten Mengenverhältnis vorgeformten Katalysatorkeimen zudosiert werden, die in einem Drehteller oder einem rotierenden schräg geneigten Mischer rollen.

Eine bevorzugte Methode der Verformung besteht darin, daß das Katalysatorpulver mit konstanter Dosiergeschwindigkeit einem vorgeformten inerten Träger zugeführt wird, der in einem rotierenden Behälter bewegt und mit einem flüssigen, benetzenden Bindemittel kontinuierlich befeuchtet wird. Als Bindemittel wird Wasser bevorzugt. Geeignete Träger sind kugelförmig vorgeformtes $Al_2O_3$ und Magnesium-Aluminiumsilikat sowie übliche keramische Massen mit Teilchendurchmessern von 1,5-5 mm. Die Menge der aktiven Masse auf dem Träger beträgt im allgemeinen 20-250 Gew.%, bezogen auf das Trägergewicht. Bevorzugt sind Mengen von 50 bis 200 Gew.%, bezogen auf das Trägergewicht.

Das erfindungsgemäße Verfahren wird im allgemeinen bei Drücken zwischen 1 und 5 bar und bei Temperaturen zwischen 200 und 400 °C vorteilhaft bei Temperaturen von 250 °C bis 330 °C durchgeführt. Die Verweilzeiten liegen zwischen 0,5 und 10 Sekunden, vorteilhaft zwischen 1 und 6 Sekunden. Als Oxidationsmittel wird im allgemeinen Luft eingesetzt, der Sauerstoff kann jedoch auch in anderer Form, z. B. als Rein-Sauerstoff dem Reaktor zugeführt werden. Die Oxidation wird vorteilhaft in Gegenwart von Inertgasen insbesondere von Wasserdampf durchgeführt. Der Wasserdampf kann zum Teil durch das Reaktionsabgas ersetzt werden, das nach Auswaschen und/oder Kondensation der Produkte in die Synthese zurückgeführt wird und im allgemeinen aus nicht umgesetztem Sauerstoff, Stickstoff, Kohlenoxiden und nicht umgesetztem Methacrolein besteht und entsprechend den Trennbedingungen mit Wasserdampf gesättigt ist. Das molare Verhältnis von Methacrolein : $O_2$ : $H_2O$ : inerte Gase beträgt im allgemeinen 1 : 1-6 : 1-20 : 4-50, vorzugsweise 1 : 1,5-4 : 2-10 : 6-30.

Das eingesetzte Methacrolein kann nach verschiedenen Verfahren hergestellt sein, z. B. durch Gasphasenoxidation von tert. Butylalkohol, Isobutylen oder $C_4$-Gemischen oder durch Kondensation von Propionaldehyd mit Formaldehyd. Bevorzugt wird technisches Methacrolein eingesetzt, das durch Kondensation von Propionaldehyd mit Formaldehyd in Gegenwart von Salzen, sek.-Amine oder mit Aminalen in Gegenwart von Säuren in wäßriger Lösung erhalten wurde.

Technische Qualitäten haben im allgemeinen einen Reinheitsgrad von 94-98 % und enthalten neben nicht umgesetztem Propionaldehyd in geringen Mengen organische Amine, wie Diethylamin oder Diethanolamin, Methylpentenal und Dimere des Methacroleins. Bedingt durch eine Rückführung des nicht umgesetzten Methacroleins kann das Synthesegasgemisch auch geringe Mengen leichter flüchtiger Nebenprodukte des erfindungsgemäßen Verfahrens enthalten. Die Reinheitsangaben beziehen sich auf wasserfreies Rohmethacrolein, im allgemeinen enthält das technische Methacrolein bis zu 3,5 Gew.% Wasser.

Die Umsetzung des Methacroleins kann in Wirbelschichtreaktoren durchgeführt werden. Vorzugsweise arbeitet man aber in sogenannten Rohrbündelreaktoren an fest angeordneten Katalysatoren. Zur Vermeidung von örtlichen Überhitzungen kann die Katalysatoraktivität so verändert werden, daß sie im Reaktionsrohr in Strömungsrichtung stetig oder in Stufen ansteigt. Geeignete Maßnahmen sind beispielsweise eine Verdünnung des geformten Katalysators mit wenigen aktiven oder inaktiven Katalysator- oder Trägerformlingen oder der Einsatz von zwei oder mehr Katalysatoren mit unterschiedlicher Aktivität und/oder Selektivität.

Die Isolierung der Methacrylsäure aus den Reaktionsgasen kann in an sich üblicher Weise durchgeführt werden. Im allgemeinen wird das Reaktionsabgas indirekt oder direkt gekühlt und mit Wasser gewaschen. Aus der erhaltenen wäßrigen Lösung kann die Methacrylsäure und ggf. Essig, Malein- und Acrylsäure mit geeigneten Lösungsmitteln, wie Methylmethacrylat, extrahiert und entweder direkt einer Veresterung mit Alkanolen zugeführt oder destillativ vom Extraktionsmittel und den Nebenprodukten getrennt werden. Das nicht umgesetzte Methacrolein kann aus dem wäßrigen Kondensat mit Wasserdampf ausgetrieben und ggf. zusammen mit nicht abgetrennten Wasseranteilen und leicht flüchtigen Nebenprodukten wieder der Synthese zugeführt werden.

Die erfindungsgemäßen Katalysatoren zeigen auch bei anderen Oxidationsverfahren, z. B. der Oxidation von Acrolein zu Acrylsäure oder der Oxidation von substituierten Toluolderivaten zu substituierten Benzaldehyden und Benzoesäuren gute Wirksamkeit und Selektivität.

In den folgenden Beispielen werden Gewichtsteile angegeben. Es wird ein Methacrolein mit einem Reinheitsgrad von 95 bis 98 Gew.% eingesetzt, das neben Propionaldehyd geringe Mengen an sek. Aminen und Nebenprodukten der Methacroleinsynthese auf Basis Propionaldehyd/Formaldehyd enthielt.

## Beispiel 1

### A. Herstellung des Katalysators

212 Teile Ammoniumheptamolybdat mit einem K-Gehalt von 220 ppm (parts per million), 22,6 Teile Phosphorsäure (85 %ig) 11,7 Teile Ammoniummetavanadat, 7,1 Teile Niobpentoxid, 39 Teile Caesiumnitrat, 12,08 Teile Kupfernitrat, 2 Teile Rhodium(III)chloridhydrat werden nacheinander unter Rühren in 1 200 Teilen Wasser bei 60 °C gelöst. Die Lösung wird auf dem Wasserbad eingedickt und der Rückstand bei 130 °C 12 Stunden getrocknet. Die Trockenmasse wird zerkleinert und nach Zusatz von 2 Gew.% Graphitpulver zu 3 × 3 mm Tabletten verpreßt.

100 ml Tabletten wurden in einem U-förmigen Reaktionsrohr von 15 mm freiem Durchmesser, das durch ein Salzbad auf 450 °C erhitzt ist, bei einem Luftdurchsatz von 8 Nl/h zwei Stunden calciniert. Der erhaltene Katalysator A hat die Zusammensetzung $Mo_{12}V_1P_{1,96}Nb_{0,53}Cs_{1,95}Cu_{0,5}Rh_{0,1}O_x$.

B. Oxidation von Methacrolein zu Methacrylsäure

85 ml Katalysator werden in ein $V_2$A-Rohr einer lichten Weite von 15 mm eingefüllt. Man heizt im Salzbad auf 260 °C und beschickt mit 85,2 Nl/h einer auf die Badtemperatur vorgeheizten Gasmischung aus 4 Vol.% Methacrolein, 65 Vol.% Luft und 31 Vol.% Wasserdampf.

Anschließend wird die Badtemperatur langsam auf 314 °C erhöht. Nach 200 Stunden beträgt der Methacroleinumsatz 73,8 Mol.%, die Ausbeute an Methacrylsäure 66,5 Mol.% und die Selektivität 90,1 Mol.%. Die Ausbeuten an Nebenprodukten betragen 1,7 Mol.% Essigsäure, etwa 0,1 Mol.% Acrylsäure, ca. 0,4 Mol.% Maleinsäure, etwa 0,4 Mol.% an sonstigen Nebenprodukten, wie Formaldehyd, Acrolein, Aceton, Acetaldehyd, Benzaldehyd, Ameisensäure und 4,8 Mol.% Kohlenoxide. Wird eine Katalysatormenge von 50 ml eingesetzt und die Badtemperatur auf 320 °C erhöht, so beträgt der Umsatz 60,6 % und die Selektivität 88,1 Mol.%.

Beispiel 2

Es wird ein Katalysator B nach den Angaben im Beispiel 1 mit dem Unterschied hergestellt, daß kein $Nb_2O_3$ zugesetzt wird. Bei einer Badtemperatur von 300 °C wird dann nach 240 Betriebsstunden unter den Beispiel 1 unter B angegebenen Bedingungen ein Umsatz von 80,6 Mol.%, eine Ausbeute an Methacrylsäure von 70 Mol.% und eine Methacrylsäureselektivität von 86,8 Mol.% erreicht.

Beispiel 3

Nach den Angaben in Beispiel 1 wird ein Katalysator der Zusammensetzung $Mo_{12}V_1P_1Cs_1Cu_{0,5}Nb_1Rh_{0,1}O_x$ hergestellt und wie in Beispiel 1 B angegeben getestet. Bei einer Badtemperatur von 300 °C wird ein Umsatz von 77,9 Mol.% und eine Selektivität der Methacrylsäurebildung von 81,9 Mol.% erreicht. Bei 315 °C beträgt der Umsatz 88,1 Mol.% und die Selektivität der Acrylsäurebildung 80,2 Mol.%.

Vergleichsversuche a bis c zu Beispiel 3

Es werden Katalysatoren entsprechend Beispiel 3 hergestellt mit dem Unterschied, daß jeweils eine der Komponenten Cu, Rh, V weggelassen wird. Die Zusammensetzung und Testergebnisse unter den Bedingungen des Beispiels 1 B sind in der folgenden Tabelle 1 zusammengefaßt.

Tabelle 1

| Versuch | Katalysator | Badtemperatur °C | Umsatz Mol.% | Selektivität Mol.% |
|---|---|---|---|---|
| a | $Mo_{12}P_1V_1Nb_1Cs_1Cu_{0,5}O_x$ | 314 | 46,1 | 68,7 |
| b | $Mo_{12}P_1V_1Nb_1Cs_1Rh_{0,1}O_x$ | 300 | 45,5 | 81,7 |
| c | $Mo_{12}P_1Nb_1Cs_1Cu_{0,5}Rh_{0,1}O_x$ | 300 | 53,9 | 80,1 |

Beispiel 4

212 Teile Ammoniumheptamolybdat mit 220 ppm Kalium, 22,6 Teile Phosphorsäure (85 %ig) 11,7 Teile Ammoniummetavanadat, 7,1 Teile Niobpentoxid, 39 Teile Caesiumnitrat, 12,08 Teile Kupfernitrat, 2 Teile Rhodium(III)chloridhydrat werden nacheinander unter Rühren in 1 200 Teilen Wasser bei 60 °C gelöst. Die Lösung wird auf dem Wasserbad eingedickt und der Behälter bei 130 °C 12 Stunden getrocknet. Die Trockenmasse wird in einem Drehrohrofen bei einem Luftddurchsatz von 8 Nl/h auf 450 °C aufgeheizt und anschließend 2 Stunden bei 450 °C calciniert. Die erhaltne Oxidmasse wird auf eine Teilchengröße von kleiner 50 µm zerkleinert. 150 Teile des erhaltenen Pulvers werden auf 145 Teile Steatitkugeln eines Durchmessers von 3 mm in der Weise aufgebracht, daß das Pulver mit konstanter Dosiergeschwindigkeit dem kontinuierlich mit Wasser befeuchteten, in einer Dragiertrommel stark bewegten Träger zudosiert wird. Nach Trocknung in der Dragiertrommel bei 70 °C mit 100 °C heißer Luft wird ein abriebfester Schalenkatalysator erhalten.

Über 85 ml des Katalysators werden in einem Salzbadreaktor 85 Nl/h einer Gasmischung aus 5 Vol.-% Methacrolein, 65 Vol.% Luft und 30 Vol.% Wasserdampf geleitet. Bei einer Badtemperatur von 340 °C wird ein Umsatz von 50,1 Mol.%, eine Ausbeute an Methacrylsäure von 44 Mol.% und eine Selektivität von 89,8 Mol.% erreicht.

Beispiel 5 bis 14

Nach den Angaben in Beispiel 1 A werden Katalysatoren mit verschiedenen Anteilen Rhodium, Kupfer und Phosphor hergestellt und unter den Bedingungen von Beispiel 1 B getestet. Die Testergebnisse sind der folgenden Tabelle 2 zusammengestellt.

Tabelle 2

| Beispiel | Katalysatorzusammensetzung | Badtemperatur °C | Umsatz Mol.% | Selektivität Mol.% |
|---|---|---|---|---|
| 5 | $Mo_{12}P_2V_1Nb_1Cs_1Cu_{0,5}Rh_{0,2}O_x$ | 318 | 58,7 | 84,2 |
| 6 | $Mo_{12}P_2V_1Nb_{0,5}Cs_1K_{0,5}Cu_{0,5}Rh_{0,1}O_x$ | 328 | 57 | 88,2 |
| 7 | $Mo_{12}P_2V_1Nb_{0,5}Cs_1Rb_1Cu_{0,5}Rh_{0,1}O_x$ | 314 | 74 | 88,2 |
| 8 | $Mo_{12}P_2V_1Nb_{0,5}Cs_2Cu_{0,5}Rh_{0,05}O_x$ | 314 | 74 | 88,2 |
| 9 | $Mo_{12}P_3V_1Nb_{0,5}Cs_2Cu_{0,5}Rh_{0,1}O_x$ | 326 | 46,4 | 87,2 |
| 10 | $Mo_{12}P_2V_1Cs_1Cu_{0,25}Rh_{0,2}Cr_{0,05}Fe_{0,5}O_x$ | 298 | 79,1 | 82,7 |
| 11 | $Mo_{12}P_2V_1Cs_1Cu_{0,25}Rh_{0,2}Nb_{0,2}Cr_{0,5}Fe_{0,05}O_x$ | 300 | 81,7 | 82,3 |
| 12 | $Mo_{12}P_2V_1Cs_1Cu_{0,5}Rh_{0,1}Nb_{0,5}Cr_{0,05}Fe_{0,5}Li_{0,5}O_x$ | 318 | 66,1 | 83,7 |
| 13 | $Mo_{12}P_2V_1Cs_1Cu_{0,5}Rh_{0,1}Nb_{0,5}Cr_{0,05}Fe_{0,5}Li_{0,25}Na_{0,25}O_x$ | 310 | 45,7 | 88,9 |
| 14 (*) | $Mo_{12}P_2V_1Cs_1Cu_{0,5}Rh_{0,1}Nb_{0,5}Cr_{0,05}Fe_{0,5}Li_{0,25}Na_{0,25}O_x$ | 310 | 45,7 | 88,9 |

(*) Bei Beispiel 14 wurden 85 ml Katalysator beschickt mit 58,3 Nl Gas/h der Zusammensetzung : 5,8 Vol.% Methacrolein, 5,4 Vol.% $O_2$, 42,9 Vol.% Wasserdampf, 46,2 Vol.% Stickstoff.

Beispiel 15

Zur Prüfung der Lebensdauer der erfindungsgemäßen Katalysatoren wurde ein Katalysator der Zusammensetzung $Mo_{12}V_1P_{1,96}Nb_{0,53}Cs_{1,95}Cu_{0,5}Rh_{0,1}O_x$ nach der Vorschrift des Beispiels 1 hergestellt. 85 ml des Katalysators wurden, wie im Beispiel 1B angegeben, auf eine Badtemperatur von 318 °C aufgeheizt und unter Konstanthaltung der Badtemperatur 1 010 Stunden getestet. Die Testergebnisse nach verschiedenen Betriebszeiten sind in der folgenden Tabelle 3 zusammengefaßt. Es ist kein Abklingen des Umsatzes und der Selektivität innerhalb der Testzeit zu beobachten.

Tabelle 3

| Betriebszeit Stunden | Umsatz Mol.% | Ausbeute Methacrylsäure Mol.% | Selektivität Mol.% |
|---|---|---|---|
| 96 | 74,1 | 64,5 | 87 |
| 360 | 74 | 63,4 | 85,6 |
| 720 | 74,3 | 64,9 | 87,4 |
| 1 010 | 74,2 | 63,7 | 85,9 |

**Ansprüche**

1. Oxidationskatalysator, dessen katalytisch aktive Komponente die allgemeine Formel

$$Mo_{12}P_aRh_bCu_cV_dCs_eX_fY_gZ_hO_x$$

hat, in der
X für Cr und/oder Fe
Y für Nb
Z für Na, Li, K und/oder Rb,
a für 0,1-4,
b für 0,001-1,

c für 0,05-2,
d für 0,05-4,
e für 0,1-5,
f für 0-2,
g für 0-3,
h für 0-2 und
x für die Anzahl der zur Absättigung der Valenzen der anderen Bestandteile erforderlichen Sauerstoffatome
stehen.

2. Verfahren zur Herstellung von Methacrylsäure durch Gasphasenoxidation von Methacrolein mit sauerstoff- und wasserdampfenthaltenden Gasmischungen an Katalysatoren, die Molybdän, Phosphor und Vanadin enthalten, dadurch gekennzeichnet, daß die Umsetzung an einem Oxidationskatalysator gemäß Anspruch 1 durchgeführt wird.

**Claims**

1. An oxidation catalyst whose catalytically active component has the general formula

$$Mo_{12}P_aRh_bCu_cV_dCs_eX_fY_gZ_hO_x$$

where
X is Cr and/or Fe,
Y is Nb,
Z is Na, Li, K and/or Rb,
a is 0.1-4,
b is 0.001-1,
c is 0.05-2,
d is 0.05-4,
e is 0.1-5,
f is 0-2,
g is 0-3,
h is 0-2, and
x is the number of oxygen atoms required to saturate the valencies of the other constituents.

2. A process for the preparation of methacrylic acid by gas phase oxidation of methacrolein with a gas mixture, containing oxygen and steam, over a catalyst containing molybdenum, phosphorus and vanadium, wherein the reaction is carried out over an oxidation catalyst as claimed in claim 1.

**Revendications**

1. Catalyseur d'oxydation dont le composant à activité catalytique a la formule générale

$$Mo_{12}P_aRh_bCu_cV_dCs_eX_fY_gZ_hO_x$$

dans laquelle
X représente Cr et/ou Fe
Y représente Nb
Z représente Na, Li, K et/ou Rb,
a représente 0,1-4,
b représente 0,001-1,
c représente 0,05-2,
d représente 0,05-4,
e représente 0,1-5,
f représente 0-2,
g représente 0-3,
h représente 0-2 et
x est le nombre d'atomes d'oxygène nécessaire pour saturer les valences des autres composants.

2. Procédé de préparation d'acide méthacrylique par oxydation en phase gazeuse de methacroléine avec des mélanges de gaz contenant de l'oxygène et de la vapeur d'eau, sur des catalyseurs contenant du molybdène, phosphore et vanadine, caractérisé par le fait que la réaction est effectuée sur un catalyseur d'oxydation selon la revendication 1.